# EUROPEAN PATENT SPECIFICATION

(11) **EP 0 871 764 B1**
(45) Date of publication and mention of the grant of the patent: **19.02.2003**
(21) Application number: 96938085.6
(22) Date of filing: 05.11.1996
(51) Int. Cl.: C12Q 1/48, G01N 33/542

(54) **SCREENING METHOD**
SCREENING VERFAHREN
PROCEDE D'EVALUATION RAPIDE

(30) Priority: 15.12.1995 GB 9525703; 15.12.1995 GB 9525705
(43) Date of publication of application: 21.10.1998
(73) Proprietor: Novartis AG, 4056 Basel (CH); Novartis-Erfindungen Verwaltungsgesellschaft m.b.H., 1235 Wien (AT)
(72) Inventor: HEMMINGS, Brian, Arthur, CH-4126 Bettingen (CH); FRECH, Matthias, CH-4056 Basle (CH)
(74) Representative: Becker, Konrad
(86) International application number: EP9604814
(87) International publication number: WO97022717

(56) References cited:
- DATABASE CHEMABS CHEMICAL ABSTRACTS SERVICE, COLUMBUS, OHIO, US MICHALAK, K. ET AL: "Interaction of erythrocyte spectrin with some nonbilayer phospholipids" XP002030409 & GEN. PHYSIOL. BIOPHYS. (1994), 13(1), 57-62 CODEN: GPBIE2;ISSN: 0231-5882, 1994,
- BIOCHEM. BIOPHYS. RES. COMMUN. (1995), 216(2), 526-34 CODEN: BBRCA9;ISSN: 0006-291X, 1995, XP002030406 KONISHI, HIROAKI ET AL: "Molecular cloning and characterization of a new member of the RAC protein kinase family: association of the pleckstrin homology domain of three types of RAC protein kinase with protein kinase C subspecies and.beta..gamma. subunits of G proteins"
- NATURE, vol. 363, 27 May 1993, pages 309-310, XP002030407 RICHARD J. HASLAM: "Pleckstrin domain homology" cited in the application
- PROCEEDINGS OF THE NATIONAL ACADEMY OF SCIENCES U.S.A., vol. 88, May 1991, pages 4171-4175, XP002030408 PAMELA F. JONES: "Molecular cloning and identification of a serine/threonine protein kinase of the second-messenger subfamily." cited in the application

## Description

The present invention concerns a method for screening for compounds which are potential modulators of signalling pathways involving the serine/threonine kinase RAC and to the use of the pleckstrin homology domain of RAC-PK in such a method.

The RAC subfamily of serine/threonine protein kinases [Jones, *et al.* (1991) *Proc. Natl. Acad. Sci. U. S. A.* **88,** 4171-4175; Jones, *et al.* (1991) *Cell Reg.* **2,** 1001-1009], which are closely related to the cAMP-dependant protein kinase (PKA) and Ca²⁺/phospholipid dependant protein kinase (PKC) families in their kinase domain, contain an amino-terminal pleckstrin homology (PH) domain [Haslam, *et al.* (1993) *Nature* **363,** 309-310]. The human RAC-PKα isoform is the protooncogenic form of *v-akt*, which encodes a complete RAC-PK with the addition of a truncated *gag* sequence at the amino-terminus providing a myristylation sequence.

The PH domain was originally identified as an internal repeat, present at the amino and carboxy-termini of pleckstrin, a 47 kDa protein which is the major PKC substrate in activated platelets [Tyers, *et al.* (1988) *Nature* **333,** 470-473]. The superfamily of PH domain containing molecules consists of over 60 members, including serine/threonine protein kinases (RAC-PK, Nrk, βARK, PKCµ), tyrosine protein kinases (Btk, Tec, Itk), GTPase regulators (ras-GAP, ras-GRF, Vav, SOS, BCR), all known mammalian phospholipase Cs, cytoskeletal proteins (β-spectrin, AFAP-110, syntrophin), "adapter" proteins (GRB-7, 3BP2) and kinase substrates (pleckstrin, IRS-1).

While the PH domain structure has been solved for β-spectrin, dynamin and pleckstrins amino-terminal domain, its precise function remains unclear. The presence of PH domains in many signalling and cytoskeletal proteins implicates it in mediating protein-protein interactions. Indeed, the PH domain of the β-adrenergic receptor kinase (βARK) appears partly responsible for its binding to the βγ-subunits of the heterotrimeric G-proteins associated with the β-adrenergic receptor, while the PH domain of the Bruton's tyrosine kinase (Btk) appears to mediate an interaction with PKC. Several PH domains have been shown to be able to bind phosphatidyl-inositol-(4,5)-bisphosphate *in vitro,* although weakly.

The molecular cloning of another member of the RAC protein kinase family, namely RAC-PKγ, was disclosed in Konishi et al. (1995), *Biochem. Biophys. Res. Commun.* 216(2), 526-534. It was found that RAC-PKγ was highly related to the previously identified family members. Further analysis revealed that the PH domain of the RAC-PK subtypes associates with both protein kinase C subspecies and the β and γ subunits of G proteins indicating that the RAC-PK family could associate more than one protein to regulate the activity and/or intracellular distribution of this enzyme family by different ways.

Michalak, K. et al. (1994), *Gen. Physiol. Biophys.* 13(1), 57-62 dislose the interaction of erythrocyte spectrin with lysophosphatidylcholine and lysophosphatidylserine as detected by small changes of intrinsic fluorescence of spectrin.

We have now shown that the PH domain of RAC-PK binds phospholipid with high affinity, which suggests that RAC-PK my be membrane-bound *in vivo* via the PH domain. Moreover, binding of phospholipid to the RAC-PK PH domain quenches the intrinsic Tryptophan (Trp) fluorescence thereof.

### Summary of the Invention

According to a first aspect of the invention, therefore, we provide a method for screening a compound which is a candidate modulator of signal response comprising the steps of:
(a) incubating the compound with the PH domain of a signalling molecule which is capable of fluorescing;
(b) determining the phospholipid-induced modulation in the fluorescence of the PH domain, an alteration of the fluorescence in the presence of the compound being indicative of a functional interaction between the compound and the PH domain.

### Detailed Description of the Invention

The observed high affinity binding of phospholipid to the N-terminus of the PH domain of signalling molecules suggests that the PH domain functions as a membrane anchor for the molecules. Interaction of molecules with the cell membrane is believed to be important for the stable interaction thereof with membrane bound partners in signalling pathways, such that disruption of this interaction will lead to modulation of the signalling effect through the dissociation of the signalling molecule from the cell membrane. The modulation could be either down-regulating, for example if the otherwise stable interaction of the molecule with membrane-bound partners is a stimulatory interaction, or up-regulating, in the event that the interaction is an inhibitory interaction.

Accordingly, compounds which affect the binding of the PH domain to phospholipids present in the cell membrane are potential modulators of signal response.

Such compounds could be agents which cause a conformational change in the PH domain, either increasing or decreasing its affinity for phospholipid, or compounds which compete directly for binding of the PH domain to the cell membrane, for example phospholipid molecules or inositol phosphates.

The effect on the PH domain of the molecule may be direct or indirect. In other words, the compound may interact directly with the PH domain to alter its affinity for membrane phospholipid, or it may exert its effect without direct interaction with the PH domain, for example by interacting with another domain of the molecule and thereby influencing the PH domain, for example causing a conformational change. Compounds which have a direct effect on the PH domain may be screened using isolated PH domain, whereas compounds having an indirect effect thereon should be screened using intact the signalling molecule.

Signalling molecules according to the invention comprise all molecules involved in signalling pathways which possess a PH domain, although the method is clearly only practicable with those PH domains having a fluorescent group in the phospholipid binding amino terminus. Such molecules are characterised by a trp residue, homologous to Trp²² in RAC-PK.

Preferably, the method of the invention is applied to protein kinases and their substrates, which are implicated in intracellular signalling and growth control. Advantageously, the method is applicable to RAC kinase and its PH domain, which shows a high affinity for phospholipid and fluoresces via Trp²² in a phospholipid-binding dependent manner.

Isolated PH domain for use in the present invention may be prepared as set forth in by recombinant expression employing an extension of at least three hydrophilic amino acids on the C-terminus of the domain. It is found that the yield and solubility of the domain in bacterial cell culture is thereby increased.

Preferably, between three and ten additional amino acid residues are present at the C-terminal end of the domain. Advantageously, three to six residues are added. In general, the greater the number of residues present, the greater the increase in hydrophilicity conferred thereby. However, this must be balanced by the clearly advantageous avoidance of an excessively long addition to the gene product.

Any hydrophilic amino acid is suitable for use in the present invention. In general, hydrophilic amino acids possess polar R-groups which improve the solubility thereof in water. Such amino acids may possess uncharged R-groups, such as glycine, serine or threonine, or charged R-groups, such as aspartic acid or glutamic acid, which are negatively charged (acidic) and lysine or arginine, which are positively charged (basic). Preferred, however, are basic amino acids such as lysine, arginine and histidine. Most preferred is lysine.

Where more than one additional amino acid is used, the additional amino acids may be the same or different. For example, it is possible to use three lysines. Alternatively, two lysines and an arginine residue may be combined.

The expression system for use in the present invention is in essence a conventional expression system which is modified by standard methodology to conform to the requirements set forth herein. In practice, the invention may be effected starting with any expression system known in the art which entail the intracellular production of a heterologous polypeptide in a bacterial host cell. The bacterial host cell may be any bacterial cell known for use in the production of heterologous polypeptides, but *E. coli* and *B. subtilis* cells are preferred. *E. Coli* is especially preferred.

Typically, the bacterial host cell is transformed with an expression vector comprising a coding sequence encoding the heterologous polypeptide, to which the requisite number of nucleotides encoding the hydrophilic amino acids to be added to the C-terminus have been appended. Addition of nucleotides to the coding sequence may be carried out by conventional means, such as insertion of oligonucleotide linkers or site-directed mutagenesis. In an alternative embodiment, however, an expression vector may be constructed In which the required nucleotides encoding a C-terminal hydrophilic extension are already in place, upstream of a suitable stop codon, so that a sequence encoding the heterologous polypeptide may be inserted directly upstream thereof by conventional cutting and pasting with restriction enzymes.

The recovery of soluble PH domain may be further enhanced by expression thereof in the form of a fusion protein. This technique involves the expression of the domain as a fusion with a further polypeptide. Usually, only the first few amino acids of the further polypeptide are present, fused to the N-terminus of the heterologous polypeptide. The fusion is made at the DNA level taking care to preserve the correct reading frame in the domain, such that the composite gene is expressed to produce the fusion protein.

Fusions may be made with a suitable gene, which may be of natural, such as bacterial, or artificial origin. Thus, for example, a fusion may be made with a β-galactosidase or a glutathione-S-transferase (GST) gene. Preferably, however, an artificial fusion is created using an artificial gene. Advantageously, the composition of the further polypeptide encoded by the artificial gene is selected to further improve the solubility of the fusion protein expression product. Advantageously, therefore, the further polypeptide comprises hydrophilic amino acids as defined hereinbefore. The further polypeptide may comprise any reasonable number of such amino acids, but a range of three to ten is preferred, with five to ten being more preferred. Advantageously, the further polypeptide comprises about six amino acids.

Preferably, the amino acids which comprise the further polypeptide are basic amino acids. Histidine is advantageously used.

Advantageously, therefore, the expression vector is adapted to express heterologous polypeptides as fusion proteins. A number of such vectors are available commercially. Particularly useful in the context of the present invention are vectors expressing fusions with further polypeptide of a hydrophilic nature, such as the pRSET vector from Invitrogen.

An advantage of using a further polypeptide which is comprised of hydrophilic amino acids is that both ends of the fusion protein are hydrophilic in character, thus further enhancing the increased solubility of the heterologous polypeptide.

Terminally added amino acids according to the invention, including the further polypeptides added as part of fusion proteins, may be removed during or after purification of the heterologous polypeptide from the cell culture by terminal digestion, enzymatic cleavage as normally carried out with fusion proteins or by other means. In the case of enzymatic cleavage, the terminal additions will advantageously comprise cleavage sites for restriction enzymes or chemical modifying agents to permit removal of the added amino acids.

The expression vector used in the invention comprises the regulatory sequences required to achieve expression in the intended host cell. in addition, it may contain the necessary sequences required for plasmid replication in order to exist in an episomal state, or it may be designed for chromosomal integration.

Regulatory sequences will include a promoter capable of driving the sequence encoding the heterologous polypeptide. For example, strong viral promoters such as the SV40, adenovirus or hCMV promoters may be used. However, expression of soluble heterologous polypeptide may be further enhanced through the use of an inducible promoter. Inducible promoters are promoters which are inactive or only active at low levels until induced by the administration of a particular stimulus to the host cell. The main advantage thereof is that they permit culturing of the cell to the required cell density before induction of heterologous polypeptide expression, thereby avoiding placing excessive metabolic load on the cell until cell growth is complete. Numerous inducible promoters are available in the art, and in general bacterial promoters which control expression of the synthesis of non-essential nutrients are inducible. The *trp* promoter of *E. coli*, for example, is induced at low concentrations of Trp in the growth medium. The β-galactosidase (*lacZ*) promoter of *E. coli*, on the other hand, is inducible by the administration of IPTG. Where a viral promoter is used, inducibility may be achieved by means of regulation of the supply of transactivators required by certain viral promoters. The adenovirus immediate-early promoter, for example, is transactivated by the E1A gene product.

Bacterial culture is normally carried out at 37°C, and induction of an inducible promoter such as the *lacZ* promoter may be carried out at the normal culturing temperature.
However, we have found that by inducing expression at a reduced temperature both yield and solubility of the product may be increased. Preferably, therefore, induction is carried out at below 37°C. The preferred range is 20 to 33°C, with 24°C being the most preferred temperature. Preferably, the induction temperature is maintained over the period during which the heterologous polypeptide is expressed.

Since the heterologous polypeptide produced according to the invention is soluble but located intracellularly, recovery thereof requires lysis of the bacterial cell wall and recovery of the heterologous polypeptide from the cell lysate. Conventional recovery procedures may be used, typically involving removal of particulate cell residue by centrifugation and subsequent purification of the protein by chromatographic procedures. The inclusion of terminal hydrophilic amino acids may significantly alter the isoelectric point of the polypeptide, allowing enhanced affinity purification on a cation exchange column at neutral pH. Moreover, inclusion of histidine as a hydrophilic amino acid means that purification may be easily achieved on a Ni(II) affinity column by virtue of the His tag on the heterologous polypeptide. Further conventional purification techniques may be practised, such as size exclusion chromatography or affinity chromatography, in order to prepare pure heterologous polypeptide.

Where a small quantity of PH domain suffices, however, PH domain may be obtained by expressing a nucleic acid sequence encoding it in bacterial cell culture in the form of a simple fusion protein which is subsequently cleaved according to techniques known in the art. For example, amino acids 1-131 of RAC-PK, which encode the PH domain, may be expressed as a fusion with glutathione-S-transferase, subsequently cleaving the fusion protein with thrombin and isolating the domain by protein purification techniques such as FPLC. This method gives a relatively small yield of pure soluble PH domain.

RAC-PK for use in the present invention may be prepared as set forth in UK patent application 9525702.8 (Ciba-Geigy AG), filed on 15th December 1995. Alternatively, RAC-PK may be expressed in recombinant cell culture. Baculovirus vectors, specifically intended for insect cell culture, are especially preferred and are widely obtainable commercially (e.g. from Invitrogen and Clontech). Other virus vectors capable of infecting insect cells are known, such as Sindbis virus (Hahn *et al*., (1992) PNAS (USA) **89,** 2679-2683). The baculovirus vector of choice (reviewed by Miller (1988) Ann. Rev. Microbiol. **42,** 177-199) is *Autographa californica* multiple nuclear polyhedrosis virus, AcMNPV.

Typically, the heterologous gene replaces at least in part the polyhedrin gene of AcMNPV, since polyhedrin is not required for virus production. In order to insert the heterologous gene, a transfer vector is advantageously used. Transfer vectors are prepared in *E. coli* hosts and the DNA insert is then transferred to AcMNPV by a process of homologous recombination. Baculovirus techniques useful in the present invention are standard and well known in the art, and are reviewed in O'Reilly *et al*., (1994) Baculovirus expression vectors; A laboratory manual, Oxford University Press Inc., NY, USA, as well as in literature published by suppliers of commercial baculovirus kits (e.g. Pharmingen).

Fluorescence of Trp residues in a PH domain may be detected by exciting the molecule to fluoresce at the appropriate frequency and monitoring the emission. RAC-PK, for example, fluoresces at 345nm when excited at 290nm. Techniques for monitoring protein fluorescence are widely known in the art.

The invention is further described, for the purposes of illustration only, in the following examples.

### Comparative Example 1

### Expression of PH domain

The PH domain of RAC-PK is expressed in bacterial cell culture as a non-fusion protein as follows: bacteria (XL1 blue; Stratagene) are transformed with the constructs pRK-PH110 or pRK-PH131. pRK-PH110 and pRK-PH131 contain amino acids 1-110 and 1-131 of human RAC-PKα respectively. They are constructed by isolating the *Nde*I-*Pfl*MI and *Nde*I-*Alw*NI fragments from pRK-hRAC-PKα [Jones, *et al*., (1991) *Proc. Natl. Acad. Sci. U. S. A.* **88,** 4171-4175] and ligating them into the *Nde*I-*Eco*RI sites of pRK172 [McLeod *et al*, (1987) EMBO J. **6,** 729-736] using a *Pfl*MI-*Eco*RI linker [5' oligo 5'-GTG GCT GAC GGC CTC TGA G-3' (SEQ ID No:7), 3' oligo 5'-AAT TCT CAG AGG CCG TCA GCC ACA GT-3' (SEQ ID No:8)] and an *Alw*NI-*Eco*RI linker [5' oligo 5'-CTT GAT GAG-3' (SEQ ID No:9), 3' oligo 5'-AAT TCT CAT CAA GCC C-3' (SEQ ID No:10)]. General molecular biological techniques are performed as previously described [Sambrook, *et al*., (1989) *Molecular Coning: A Laboratory Manual,* 2nd Ed., Cold Spring Harbor Laboratory, Cold Spring Harbor, NY; Davis, *et al*., (1986) *Basic Methods in Molecular Biology.* Elsevier Science Publishing Co., New York, NY].

Bacteria (JM109) are grown in LB medium at 37°C in an air-shaker with rotation at 150 rpm. Induction with 0.1 mM IPTG of strains carrying the plasmids listed above is commenced when the cultures reach an O.D.₆₀₀ₙₘ of 1.0, for 0-16 hr at 37°C. Aliquots of the cultures are harvested at various times points, washed with one volume of PBS and then stored at -80°C before being analysed. Cell pellets are lysed in buffer (PBS, 1% Triton X-100, 1 mM benzamidine, 0.1 mM PMSF) using a French Press. The lysate is then centrifuged at 12000 X g to separate soluble and insoluble protein fractions. The insoluble pellet is resuspended in the above buffer. Protein in the soluble and insoluble fractions are analysed by coomassie stained SDS-PAGE gels and quantitated using the BIO-RAD protein quantification assay. Protein preparations are also subjected to Western blotting analysis [Harlow, E., and Lane, D. (1988) *Antibodies-A Laboratory Manual,* Cold Spring Harbor Laboratory, Cold Spring Harbor, NY] using a polyclonal rabbit antiserum directed against bacterially expressed full-length human RAC-PKα raised by injecting rabbits subcutaneously with RACα protein and purified by precipitation using 50% (NN₄)₂SO₄ followed by affinity chromatography on Protein-A Sepharose (Pharmacia). The secondary antibody is a horse radish peroxidase coupled anti-rabbit antibody (Amersham) which is detected using the ECL method (Amersham) by autoradiography.

This method produced significant amounts of protein (∼20 mg/l) but in a totally insoluble form.

### Example 2

### The PH domain of RAC-PK is expressed as a fusion polypeptide with a (His)₆ tag.

pRSET-PH110 and pRSET-PH131 contains in-frame fusions of amino acids 1-110 and 1-131 of human RAC-PKα, respectively, with an amino-terminal (HIS)₆-tag. They are constructed by inserting the *Nde*I*-Eco*RI fragment of pRK-PH110 or pRK-PH131, respectively, into the *Bam*HI*-Eco*RI sites of pRSET-A using a *Bam*HI*-Nde*I linker [5' oligo 5'-GAT CCG CTG GAG C-3' (SEQ ID No:13); 3' oligo 5'-TAG CTC CAG CG-3' (SEQ ID No:14)].

pRSET-PH112 contains an in-frame fusion of amino acids 1-112 of human RAC-PKα with an amino-terminal (HIS)₆-tag. It is constructed by subcloning a *Nde*I-*Pf*lMI fragment of pRK-hRAC-PKα into the BamHI-EcoRI sites of pRSET-A using a BamHI-NdeI linker (see above) and a *Pfl*MI-*Eco*RI linker [5' oligo 5'-GTG GCT GAC GGC CTC AAG AAG TGA G-3' (SEQ No:18); 3' oligo 5'-AAT TCT CAC TTC TTG AGG CCG TCA GCC ACA GT-3' (SEQ ID No:15)].

Expression conditions were as for comparative Example 1, except that bacterial strains JM109(DE3), BL21 (DE3)pLysS and BL21 (DE3)pLysE (Invitrogen) are used for the production of (HIS)₆-tagged proteins.

The pRSET constructs (pSRET-PH110, pRSET-PH112 and pRSET-PH131) produce significant amounts of protein (∼2 mg/l) that is partially (∼20%) soluble. The strain BL21(DE3)pLysE produces result indistinguishable from that of BL21(DE3)pLysS. However, the strain JM109(DE3) produced less protein that is also less soluble and not inducible to the same extent as the BL21 strains.

### Example 3

pRSET-PHQ116KKK contains an in-frame fusion of amino acids 1-116 of human RAC-PKα with an amino-terminal (HIS)₆-tag, the addition of three lysines at the carboxyl-terminus, and the internal methionine, amino acid number 63, is mutated to a glutamine. It is constructed by inserting a *Pvu*II-*Bbs*I linker [5' oligo 5'-CTG CM AAG AGG G-3' (SEQ ID No:16); 3' oligo 5'-CGC TCC GTC TTT TGC AG-3' (SEQ ID No:17)] into pRSET-PH131 digested with *Pvu*II-*Bbs*I. A *Pfl*MI-*Eco*RI linker [5' oligo 5'-GTG GOT GAC GGC CTC AAG AAG CAG AAG AAG AAG TGA G-3' (SEQ ID No:18); 3' oligo 5'-AAT TCT CAC TTC TTC TTC TGC TTC TTG AGG CCG TCA GCC ACA GT-3' (SEQ ID No:19)] is then inserted into the resultant plasmid at its *Pfl*MI-*Eco*RI sites. The C-terminal differences between the pRSET vectors employed are represented in Table 1.

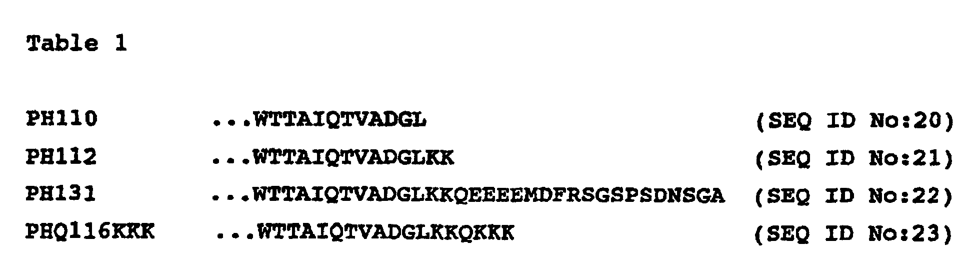

Summary of differences between pRSET expression constructs containing various carboxy-terminal elements.

Using expression conditions as for Example 3, the production from pRSET-PHQ116KKK is significant (∼5 mg/l) and almost all is soluble. Induction at 24°C as compared to 37°C also increases production and solubility. Production reaches at peak around two hours after induction at 24°C.

A large scale production (20 L) of BL21 (DE3)pLysS transformed with pRSET-PH116KKK by two hours induction at 24°C is used to purify ∼100 mg of the protein. The presence of the (HIS)₆ tag allows purification on the Ni(II) affinity column as follows: the soluble fraction of protein from BL21(DE3)pLysS cells carrying the plasmid pRSET-PHQ116KKK harvested after 2 hours of induction with 0.1 mM IPTG at 24°C and lysed in 20 mM phosphate buffer pH 7.2, 1.0 M NaCl, 1 mM benzamidine, 0.1 mM PMSF is loaded onto the column. The column, a 5 ml High-Trap metal cheating column (Pharmacia) is prepared as described by the manufacturer to contain Ni(II) ions. After loading the column with the sample it is washed sequentially with 5 bed volumes each of equilibration buffer (20 mM phosphate buffer pH 7.2, 1.0 M NaCl), ammonia buffer (20 mM phosphate buffer pH 7.2, 1.0 M NH₄Cl), low pH buffer (20 mM phosphate buffer pH 3.5, 0.5 M NaCl) and finally stripping buffer (20 mM phosphate buffer pH 7.2, 1.0 M NaCl, 50 mM EDTA). The eluted fractions are then analysed by SDS-PAGE.

This produces an approximately 50 fold purification. The high isoelectric point of the fusion protein (pl 9.6) allows the use of a cation exchange column to greatly enrich the protein a further 50 fold, as follows: the sample is equilibrated in buffer (50 mM Tris-HCl pH 7.0, 0.1 M NaCl, 2 mM EDTA, 1 mM DTT) by dialysis and loaded onto a 50 ml High-load-S cation exchange column (Pharmacia) which is equilibrated in the same buffer. Proteins are then eluted from the column with an ascending linear gradient of NaCl from 0.1 to 1.0 M in the above buffer. The column is connected to an FPLC system (Pharmacia). Collected fractions are then analysed by SDS-PAGE.

The third step is purification on a 1 I sephacryl S-300 HR gel-filtration column (Pharmacia). The sample is concentrated to 5 ml (5 mg/ml) using an ultrafiltration unit (Pharmacia) and then applied to the column equilibrated in 20 mM Tris-HCl pH 7.0, 0.2 M NaCl, 2 mM EDTA, 1 mM DTT. Protein is eluted with the same buffer and fractions are analysed by SDS-PAGE.

This third purification step on the gel-filtration column produces an apparently homogeneous preparation. The purified protein is confirmed as the protein of interest by Western blot analysis using a human RAC-PKα specific polyclonal antiserum. Amino-terminal peptide sequencing of the purified protein shows that the first 10 amino acids are identical to that of the predicted sequence.

### Example 4

### Preparation of RAC-PK PH domain as a GST fusion

General molecular biological techniques are performed as previously described [Maniatis, *et al*., (1982) *Molecular Coning: A Laboratory Manual*, Cold Spring Harbor Laboratory, Cold Spring Harbor, NY; Sambrook *et al*., (1989) *Molecular Coning: A Laboratory Manual*, p.441, 2nd Ed., Cold Spring Harbor Laboratory, Cold Spring Harbor, NY; Davis, *et al*., (1986) *Basic Methods in Molecular Biology.* Elsevier Science Publishing Co., New York, NY].

The glutathione-*S*-transferase (GST) fusion vector pGEX-2T is obtained from Dr. P Matthias (FMI, Basel, Switzerland). pRKPH131 contains amino acids 1-131 of human RAC-PKα (SEQ ID NOs. 1 and 2). It is constructed by isolating the *Nde*I-*Alw*NI fragment from pRK-hRAC-PKα [Jones, *et al*., (1991) *Proc. Natl. Acad. Sci. U. S. A.* **88**, 4171-4175] and ligating it into the *Nde*I*-Eco*RI sites of pRK172 using an *Alw*NI*-Eco*RI linker [5' oligo 5'-CTT GAT GAG-3' (SEQ ID No:3), 3' oligo 5'-AAT TCT CAT CAA GCC C-3' (SEQ ID No:4)].

pRSET-PH131 contains in-frame fusions of amino acids 1-131 of human RAC-PKα with an amino-terminal (HIS)₆-tag. It is constructed by inserting the *Nde*I*-Eco*RI fragment of pRK-pRK-PH131, into the *Bam*HI-*Eco*RI sites of pRSET-A (Invitrogen) using a *Bam*HI-*Nde*I linker [5' oligo 5'-GAT CCG CTG GAG C-3' (SEQ ID No:5); 3' oligo 5'-TAG CTC CAG CG-3' (SEQ ID No:6)].

pGEX-PH131 contains an in-frame fusion of amino acids 1-131 of human RAC-PKα with GST. It is constructed by subcloning a *Bam*HI-*Eco*RI fragment from pRSET-PH131 into the *Bam*HI*-Eco*RI sites of pGEX-2T.

The GST-RAC-PK fusion protein encoding expression vectors are expressed in *E. coli* cells follows: bacteria (JM109) are grown in LB medium at 37°C in an air-shaker with rotation at 150 rpm. Induction with 0.1 mM IPTG of strains carrying the plasmids listed above is commenced when the cultures reach an O.D.₆₀₀ₙₘ of 0.8, for 3hrs at 25°C. Cell pellets are lysed in buffer (PBS, 1% Triton X-100, 1 mM benzamidine, 0.1 mM PMSF) using a French Press. The lysate is then centrifuged at 12000 X g, +5 min., 4°C, to separate soluble and insoluble protein fractions. The GST fusion protein is precipitated or bound to glutathione coupled to agarose, washed, eluted with glutathione, treated with thrombin to remove the GST fusion peptide and concentrated on Mono-Q by FPLC according to standard methodology.

The pGEX-PH131 construct in JM109 produces soluble protein that is easily purifiable but the yield is not very high (200 µg/l).

### Example 5

### Production of RAC protein kinase

Full-length human RAC-PKα is expressed and purified from a baculovirus system. Briefly, a baculovirus is constructed by co-transfection of Sf9 cells with pVL1392-hRAC-PKα and wild-type baculovirus AcMNPV DNA, purified by limiting dilution and detected by dot-blot hybridisation. The purified virus is used to produce human RAC-PKα in Sf9 cells. The human RAC-PKα is purified by sequential anion exchange, phospho-cellulose and gel filtration chromatography.

### Example 6

### Fluorescence of RAC-PK PH domain

Purified PH domain obtained according to Example 1 is incubated at a concentration of 1.4µM in 10mM Hepes/NaOH, pH 7.0, 10mM MgCl₂, at 25°C. When excited at 290nm, using a slit width of 5.0nm and an emission slit width of 6.5nm, peak fluorescence of approximately 800 RU is observed at 345nm.

### Example 7

### Phospholipids and Inositol Phosphates reduce fluorescence

Under the same conditions as Example 3, the RAC-PK PH domain is incubated in the presence of varying concentrations of phospholipids and inositol phosphates and fluorescence is measured. Peak fluorescence remains at 345nm, but is reduced in according to the concentration of the lipid. The maximal achievable quenching, in all cases, is 30%. In the case of Ptd-Ins (3,4,5) trisphosphate, quenching of 345nm fluorescence of 100 nM RAC-PK PH domain incubated in Hepes/MgCl₂ as above ranges from nil at 0µM to virtually 100% of maximal at 6µM. From the signal reduction, the dissociation constant for the lipids and inositol phosphates can be calculated and is set out in Table 1.

**Table 1**

| Compound | Dissociation Constant (µM) |
|---|---|
| Ptd-Ins (4,5)bisphosphate | 0.40 |
| Ptd-Ins (3,4,5)trisphosphate | 0.65 |
| | |
| Ins (1,4) bisphosphate | 0.9 |
| Ins (1,4,5) trisphosphate | 1.2 |
| | |
| Ins (1) monophosphate | 7.0 |
| Ins (2) monophosphate | 8.4 |
| | |
| Ptd-Ins: Phosphatidyl-inositol (lipids) | |
| Ins: Inositol (inositol phosphates) | |

## Claims

1. A method for screening a compound which is a candidate modulator of signal response comprising the steps of:
(a) incubating the compound with the Pleckstrin Homology (PH) domain of a signalling molecule which is capable of fluorescing;
(b) determining the phospholipid-induced modulation in the fluorescence of the PH domain, an alteration of the fluorescence in the presence of the compound being indicative of a functional interaction between the compound and the PH domain.

2. A method according to claim 1 wherein the fluorescence is associated with a Tryptophan (Trp) residue located in the N-terminus of the PH domain.

3. A method according to claim 1 or claim 2 wherein the signalling molecule is a protein kinase.

4. A method according to claim 3 wherein the signalling molecule is RAC protein kinase and the fluorescent residue is Trp²².

5. A method according to any preceding claim comprising incubating the candidate modulator compound with purified PH domain.

6. A method accordion to claim 5 wherein the PH domain is produced by expressing a recombinant expression vector encoding the PH domain as a glutathione-S-transferase (GST) fusion in a bacterial bacterial host cell, subsequently cleaving the GST fusion with thrombin and purifying the PH domain.

7. A method according to claim 4 wherein the PH domain is excited at 290nm and fluorescence is detected at 345nm.

8. A method for producing the PH domain of RAC protein kinase comprising: transforming a bacterial cell with a nucleic acid encoding the PH domain of RAC protein kinase having at least 3 hydrophilic amino acids directly joined to the C-terminus of the PH domain of RAC protein kinase; and incubating said bacterial cell such that said nucleic acid is expressed.

9. A method according to claim 8 wherein the hydrophilic amino acids are the same or different and are basic amino acids.

10. A method according to claim 9 wherein the hydrophilic amino acids are lysine residues.

11. A method according to claim 10 wherein expression of the domain is induced at a temperature between 20 and 33°C.

## Patentansprüche

1. Verfahren zum Screenen einer Verbindung, die ein potentieller Modulator einer Signalantwort ist, das die folgenden Schritte umfaßt:
(a) Inkubation der Verbindung mit der Pleckstrinhomologiedomäne (PH) eines Signalmoleküls. das zur Fluoreszenz fähig ist.
(b) Bestimmung der durch Phospholipid induzierten Modulation der Fluoreszenz der PH Domäne, wobei eine Veränderung der Fluoreszenz in Gegenwart der Verbindung eine funktionelle Wechselwirkung zwischen der Verbindung und der PH Domäne anzeigt.

2. Verbindung nach Anspruch 1, worin die Fluoreszenz mit dem Tryptophanrest (Trp) assoziiert ist, der im N-Terminus der PH Domäne lokalisiert ist.

3. Verfahren nach Anspruch 1 oder Anspruch 2, worin das Signalmolekül eine Proteinkinase ist.

4. Verfahren nach Anspruch 3, worin das Signalmolekül RAC Proteinkinase ist und der Fluoreszenzrest Trp²² ist.

5. Verfahren nach einem der vorangehenden Ansprüche, das die Inkubation der potentiellen Modulatorverbindung mit einer gereinigten PH Domäne umfaßt.

6. Verfahren nach Anspruch 5, worin die PH Domäne hergestellt wird durch Expression eines rekombinanten Expressionsvektors, der die PH Domäne als Glutathion-S-transferasefusion (GST) in einer bakteriellen Wirtszelle kodiert, anschließender Spaltung der GST Fusion mit Thrombin und Reinigung der PH Domäne.

7. Verfahren nach Anspruch 4, worin die PH Domäne bei 290 nm angeregt wird und die Fluoreszenz bei 345 nm detektiert wird.

8. Verfahren zur Herstellung der PH Domäne der RAC Proteinkinase, **gekennzeichnet durch** Transformation einer bakteriellen Zelle mit einer Nukleinsäure, die die PH Domäne der RAC Proteinkinase mit mindestens 3 hydrophilen Aminosäuren kodiert, die direkt an den C-Terminus der PH Domäne der RAC Proteinkinase gebunden sind und Inkubation dieser bakteriellen Zelle, so daß die Nukleinsäure exprimiert wird.

9. Verfahren nach Anspruch 8, worin die hydrophilen Aminosäuren gleich oder verschieden sind und basische Aminosäuren sind

10. Verfahren nach Anspruch 9, worin die hydrophilen Aminosäuren Lysinreste sind.

11. Verfahren nach Anspruch 10, worin die Expression der Domäne bei einer Temperatur zwischen 20 und 33°C induziert wird.

## Revendications

1. Procédé de sélection d'un composé qui est un modulateur candidat de la réponse signal, comprenant les étapes consistant à :
(a) faire incuber le composé avec le domaine d'homologie de la pleckstrine (PH) d'une molécule signal qui est capable de fluorescence ;
(b) déterminer la modulation induite par les phospholipides de la fluorescence du domaine de PH, une altération de la fluorescence en présence du composé étant une indication d'une interaction fonctionnelle entre le composé et le domaine de PH.

2. Procédé selon la revendication 1, dans lequel la fluorescence est associée à un résidu tryptophane (Trp) situé à l'extrémité N-terminale du domaine de PH.

3. Procédé selon la revendication 1 ou la revendication 2, dans lequel la molécule signal est une protéine-kinase.

4. Procédé selon la revendication 3, dans lequel la molécule signal est la protéine-kinase RAC et le résidu fluorescent est le Trp²².

5. Procédé selon l'une quelconque des revendications précédentes, comprenant l'incubation du composé modulateur candidat avec un domaine de PH purifié.

6. Procédé selon la revendication 5, dans lequel le domaine de PH est produit en exprimant un vecteur d'expression recombinant codant pour le domaine de PH comme une fusion de glutathion-S-transférase (GST) dans une cellule hôte bactérienne, ensuite en clivant la fusion de GST avec de la thrombine, et en purifiant le domaine de PH.

7. Procédé selon la revendication 4, dans lequel le domaine de PH est excité à 290 nm et la fluorescence est détectée à 345 nm.

8. Procédé de production du domaine de PH de la protéine-kinase RAC, comprenant : la transformation d'une cellule bactérienne avec un acide nucléique codant pour le domaine de PH de la protéine-kinase RAC ayant au moins 3 acides aminés hydrophiles reliés directement à l'extrémité C-terminale du domaine de PH de la protéine-kinase RAC ; et l'incubation de ladite cellule bactérienne de telle sorte que ledit acide nucléique soit exprimé.

9. Procédé selon la revendication 8, dans lequel les acides aminés hydrophiles sont identiques ou différents, et sont des acides aminés basiques.

10. Procédé selon la revendication 9, dans lequel les acides aminés hydrophiles sont des résidus lysine.

11. Procédé selon la revendication 10, dans lequel l'expression du domaine est induite à une température comprise entre 20 et 33 °C.


### SEQUENCE LISTING

(1) GENERAL INFORMATION:
   (i) APPLICANT:
      (A) NAME: CIBA-GEIGY AG
      (B) STREET: Klybeckstr. 141
      (C) CITY: Basel
      (E) COUNTRY: Switzerland
      (F) POSTAL CODE (ZIP): 4002
      (G) TELEPHONE: +41 61 69 11 11
      (H) TELEFAX: + 41 61 696 79 76
      (I) TELEX: 962 991
   (ii) TITLE OF INVENTION: Screening Method
   (iii) NUMBER OF SEQUENCES: 23
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
(2) INFORMATION FOR SEQ ID NO: 1:
   (i) SEQUENCE CHARACTERISTICS:
      (A) LENGTH: 2610 base pairs
      (B) TYPE: nucleic acid
      (C) STRANDEDNESS: single
      (D) TOPOLOGY: linear
   (ii) MOLECULE TYPE: cDNA to mRNA
   (iii) HYPOTHETICAL: NO
   (iv) ANTI-SENSE: NO
   (vi) ORIGINAL SOURCE:
      (A) ORGANISM: Homo sapiens
   (vii) IMMEDIATE SOURCE:
      (B) CLONE: Human RAC-PK alpha
   (ix) FEATURE:
      (A) NAME/KEY: CDS
      (B) LOCATION:199..1641
   (xi) SEQUENCE DESCRIPTION: SEQ ID NO: 1:
   ATCCTGGGAC AGGGCACAGG GCCATCTGTC ACCAGGGGCT TAGGGAAGGC CGAGCCAGCC       60
   TGGGTCAAAG AAGTCAAAGG GGCTGCCTGG AGGAGGCAGC CTGTCAGCTG GTGCATCAGA      120
   GGCTGTGGCC AGGCCAGCTG GGCTCGGGGA GCGCCAGCCT GAGAGGAGCG CGTGAGCGTC      180
   GCGGGAGCCT CGGGCACC ATG AGC GAC GTG GCT ATT GTG AAG GAG GGT TGG        231
                       Met Ser Asp Val Ala Ile Val Lys Glu Gly Trp
                         1               5                  10
   CTG CAC AAA CGA GGG GAG TAC ATC AAG ACC TGG CGG CCA CGC TAC TTC        279
   Leu His Lys Arg Gly Glu Tyr Ile Lys Thr Trp Arg Pro Arg Tyr Phe
                15                  20                  25
   CTC CTC AAG AAT GAT GGC ACC TTC ATT GGC TAC AAG GAG CGG CCG CAG        327
   Leu Leu Lys Asn Asp Gly Thr Phe Ile Gly Tyr Lys Glu Arg Pro Gln
            30                  35                  40
   GAT GTG GAC CAA CGT GAG GCT CCC CTC AAC AAC TTC TCT GTG GCG CAG        375
   Asp Val Asp Gln Arg Glu Ala Pro Leu Asn Asn Phe Ser Val Ala Gln
        45                  50                  55
   TGC CAG CTG ATG AAG ACG GAG CGG CCC CGG CCC AAC ACC TTC ATC ATC        423
   Cys Gln Leu Met Lys Thr Glu Arg Pro Arg Pro Asn Thr Phe Ile Ile
    60                  65                  70                  75
   CGC TGC CTG CAG TGG ACC ACT GTC ATC GAA CGC ACC TTC CAT GTG GAG        471
   Arg Cys Leu Gln Trp Thr Thr Val Ile Glu Arg Thr Phe His Val Glu
                    80                  85                  90
   ACT CCT GAG GAG CGG GAG GAG TGG ACA ACC GCC ATC CAG ACT GTG GCT        519
   Thr Pro Glu Glu Arg Glu Glu Trp Thr Thr Ala Ile Gln Thr Val Ala
                95                 100                 105
   GAC GGC CTC AAG AAG CAG GAG GAG GAG GAG ATG GAC TTC CGG TCG GGC      567
   Asp Gly Leu Lys Lys Gln Glu Glu Glu Glu Met Asp Phe Arg Ser Gly
           110                 115                 120
   TCA CCC AGT GAC AAC TCA GGG GCT GAA GAG ATG GAG GTG TCC CTG GCC      615
   Ser Pro Ser Asp Asn Ser Gly Ala Glu Glu Met Glu Val Ser Leu Ala
       125                 130                 135
   AAG CCC AAG CAC CGC GTG ACC ATG AAC GAG TTT GAG TAC CTG AAG CTG      663
   Lys Pro Lys His Arg Val Thr Met Asn Glu Phe Glu Tyr Leu Lys Leu
   140                 145                 150                 155
   CTG GGC AAG GGC ACT TTC GGC AAG GTG ATC CTG GTG AAG GAG AAG GCC      711
   Leu Gly Lys Gly Thr Phe Gly Lys Val Ile Leu Val Lys Glu Lys Ala
                   160                 165                 170
   ACA GGC CGC TAC TAC GCC ATG AAG ATC CTC AAG AAG GAA GTC ATC GTG      759
   Thr Gly Arg Tyr Tyr Ala Met Lys Ile Leu Lys Lys Glu Val Ile Val
               175                 180                 185
   GCC AAG GAC GAG GTG GCC CAC ACA CTC ACC GAG AAC CGC GTC CTG CAG      807
   Ala Lys Asp Glu Val Ala His Thr Leu Thr Glu Asn Arg Val Leu Gln
           190                 195                 200
   AAC TCC AGG CAC CCC TTC CTC ACA GCC CTG AAG TAC TCT TTC CAG ACC      855
   Asn Ser Arg His Pro Phe Leu Thr Ala Leu Lys Tyr Ser Phe Gln Thr
       205                 210                 215
   CAC GAC CGC CTC TGC TTT GTC ATG GAG TAC GCC AAC GGG GGC GAG CTG      903
   His Asp Arg Leu Cys Phe Val Met Glu Tyr Ala Asn Gly Gly Glu Leu
   220                 225                 230                 235
   TTC TTC CAC CTG TCC CGG GAA CGT GTG TTC TCC GAG GAC CGG GCC CGC      951
   Phe Phe His Leu Ser Arg Glu Arg Val Phe Ser Glu Asp Arg Ala Arg
                   240                 245                 250
   TTC TAT GGC GCT GAG ATT GTG TCA GCC CTG GAC TAC CTG CAC TCG GAG      999
   Phe Tyr Gly Ala Glu Ile Val Ser Ala Leu Asp Tyr Leu His Ser Glu
               255                 260                 265
   AAG AAC GTG GTG TAC CGG GAC CTC AAG CTG GAG AAC CTC ATG CTG GAC      1047
   Lys Asn Val Val Tyr Arg Asp Leu Lys Leu Glu Asn Leu Met Leu Asp
               270                 275                 280
   AAG GAC GGG CAC ATT AAG ATC ACA GAC TTC GGG CTG TGC AAG GAG GGG      1095
   Lys Asp Gly His Ile Lys Ile Thr Asp Phe Gly Leu Cys Lys Glu Gly
           285                 290                 295
   ATC AAG GAC GGT GCC ACC ATG AAG ACC TTT TGC GGC ACA CCT GAG TAC      1143
   Ile Lys Asp Gly Ala Thr Met Lys Thr Phe Cys Gly Thr Pro Glu Tyr
   300                 305                 310                 315
   CTG GCC CCC GAG GTG CTG GAG GAC AAT GAC TAC GGC CGT GCA GTG GAC      1191
   Leu Ala Pro Glu Val Leu Glu Asp Asn Asp Tyr Gly Arg Ala Val Asp
                   320                 325                 330
   TGG TGG GGG CTG GGC GTG GTC ATG TAC GAG ATG ATG TGC GGT CGC CTG      1239
   Trp Trp Gly Leu Gly Val Val Met Tyr Glu Met Met Cys Gly Arg Leu
               335                 340                 345
   CCC TTC TAC AAC CAG GAC CAT GAG AAG CTT TTT GAG CTC ATC CTC ATG      1287
   Pro Phe Tyr Asn Gln Asp His Glu Lys Leu Phe Glu Leu Ile Leu Met
           350                 355                 360
   GAG GAG ATC CGC TTC CCG CGC ACG CTT GGT CCC GAG GCC AAG TCC TTG      1335
   Glu Glu Ile Arg Phe Pro Arg Thr Leu Gly Pro Glu Ala Lys Ser Leu
       365                 370                 375
   CTT TCA GGG CTG CTC AAG AAG GAC CCC AAG CAG AGG CTT GGC GGG GGC      1383
   Leu Ser Gly Leu Leu Lys Lys Asp Pro Lys Gln Arg Leu Gly Gly Gly
   380                 385                 390                 395
   TCC GAG GAC GCC AAG GAG ATC ATG CAG CAT CGC TTC TTT GCC GGT ATC      1431
   Ser Glu Asp Ala Lys Glu Ile Met Gln His Arg Phe Phe Ala Gly Ile
                   400                 405                 410
   GTG TGG CAG CAC GTG TAC GAG AAG AAG CTC AGC CCA CCC TTC AAG CCC      1479
   Val Trp Gln His Val Tyr Glu Lys Lys Leu Ser Pro Pro Phe Lys Pro
               415                 420                 425
   CAG GTC ACG TCG GAG ACT GAC ACC AGG TAT TTT GAT GAG GAG TTC ACG      1527
   Gln Val Thr Ser Glu Thr Asp Thr Arg Tyr Phe Asp Glu Glu Phe Thr
           430                 435                 440
   GCC CAG ATG ATC ACC ATC ACA CCA CCT GAC CAA GAT GAC AGC ATG GAG        1575
   Ala Gln Met Ile Thr Ile Thr Pro Pro Asp Gln Asp Asp Ser Met Glu
       445                 450                 455
   TGT GTG GAC AGC GAG CGC AGG CCC CAC TTC CCC CAG TTC TCC TAC TCG        1623
   Cys Val Asp Ser Glu Arg Arg Pro His Phe Pro Gln Phe Ser Tyr Ser
   460                 465                 470                 475
   GCC AGC AGC ACG GCC TGA GGCGGCGGTG GACTGCGCTG GACGATAGCT               1671
   Ala Ser Ser Thr Ala  *
                   480
   TGGAGGGATG GAGAGGCGGC CTCGTGCCAT GATCTGTATT TAATGGTTTT TATTTCTCGG      1731
   GTGCATTTGA GAGAAGCCAC GCTGTCCTCT CGAGCCCAGA TGGAAAGACG TTTTTGTGCT      1791
   GTGGGCAGCA CCCTCCCCCG CAGCGGGGTA GGGAAGAAAA CTATCCTGCG GGTTTTAATT      1851
   TATTTCATCC AGTTTGTTCT CCGGGTGTGG CCTCAGCCCT CAGAACAATC CGATTCACGT      1911
   AGGGAAATGT TAAGGACTTC TACAGCTATG CGCAATGTGG CATTGGGGGG CCGGGCAGGT      1971
   CCTGCCCATG TGTCCCCTCA CTCTGTCAGC CAGCCGCCCT GGGCTGTCTG TCACCAGCTA      2031
   TCTGTCATCT CTCTGGGGCC CTGGGCCTCA GTTCAACCTG GTGGCACCAG ATGCAACCTC      2091
   ACTATGGTAT GCTGGCCAGC ACCCTCTCCT GGGGGTGGCA GGCACACAGC AGCCCCCCAG      2151
   CACTAAGGCC GTGTCTCTGA GGACGTCATC GGAGGCTGGG CCCCTGGGAT GGGACCAGGG      2211
   ATGGGGGATG GGCCAGGGTT TACCCAGTGG GACAGAGGAG CAAGGTTTAA ATTTGTTATT      2271
   GTGTATTATG TTGTTCAAAT GCATTTTGGG GGTTTTTAAT CTTTGTGACA GGAAAGCCCT      2331
   CCCCCTTCCC CTTCTGTGTC ACAGTTCTTG GTGACTGTCC CACCGGAGCC TCCCCCTCAG      2391
   ATGATCTCTC CACGGTAGCA CTTGACCTTT TCGACGCTTA ACCTTTCCGC TGTCGCCCCA      2451
   GGCCCTCCCT GACTCCCTGT GGGGGTGGCC ATCCCTGGGC CCCTCCACGC CTCCTGGCCA      2511
   GACGCTGCCG CTGCCGCTGC ACCACGGCGT TTTTTTACAA CATTCAACTT TAGTATTTTT      2571
   ACTATTATAA TATAATATGG AACCTTCCCT CCAAATTCT                             2610
(2) INFORMATION FOR SEQ ID NO: 2:
   (i) SEQUENCE CHARACTERISTICS:
      (A) LENGTH: 481 amino acids
      (B) TYPE: amino acid
      (D) TOPOLOGY: linear
   (ii) MOLECULE TYPE: protein
   (xi) SEQUENCE DESCRIPTION: SEQ ID NO: 2:
   Met Ser Asp Val Ala Ile Val Lys Glu Gly Trp Leu His Lys Arg Gly
    1               5                  10                  15
   Glu Tyr Ile Lys Thr Trp Arg Pro Arg Tyr Phe Leu Leu Lys Asn Asp
               20                  25                  30
   Gly Thr Phe Ile Gly Tyr Lys Glu Arg Pro Gln Asp Val Asp Gln Arg
           35                  40                  45
   Glu Ala Pro Leu Asn Asn Phe Ser Val Ala Gln Cys Gln Leu Met Lys
       50                  55                  60
   Thr Glu Arg Pro Arg Pro Asn Thr Phe Ile Ile Arg Cys Leu Gln Trp
    65                  70                  75                  80
   Thr Thr Val Ile Glu Arg Thr Phe His Val Glu Thr Pro Glu Glu Arg
                   85                  90                  95
   Glu Glu Trp Thr Thr Ala Ile Gln Thr Val Ala Asp Gly Leu Lys Lys
               100                 105                 110
   Gln Glu Glu Glu Glu Met Asp Phe Arg Ser Gly Ser Pro Ser Asp Asn
           115                 120                 125
   Ser Gly Ala Glu Glu Met Glu Val Ser Leu Ala Lys Pro Lys His Arg
       130                 135                 140
   Val Thr Met Asn Glu Phe Glu Tyr Leu Lys Leu Leu Gly Lys Gly Thr
   145                 150                 155                 160
   Phe Gly Lys Val Ile Leu Val Lys Glu Lys Ala Thr Gly Arg Tyr Tyr
   165 170 175
   Ala Met Lys Ile Leu Lys Lys Glu Val Ile Val Ala Lys Asp Glu Val
   180 185 190
   Ala His Thr Leu Thr Glu Asn Arg Val Leu Gln Asn Ser Arg His Pro
   195 200 205
   Phe Leu Thr Ala Leu Lys Tyr Ser Phe Gln Thr His Asp Arg Leu Cys
   210 215 220
   Phe Val Met Glu Tyr Ala Asn Gly Gly Glu Leu Phe Phe His Leu Ser
   225 230 235 240
   Arg Glu Arg Val Phe Ser Glu Asp Arg Ala Arg Phe Tyr Gly Ala Glu
   245 250 255
   Ile Val Ser Ala Leu Asp Tyr Leu His Ser Glu Lys Asn Val Val Tyr
   260 265 270
   Arg Asp Leu Lys Leu Glu Asn Leu Met Leu Asp Lys Asp Gly His Ile
   275 280 285
   Lys Ile Thr Asp Phe Gly Leu Cys Lys Glu Gly Ile Lys Asp Gly Ala
   290 295 300
   Thr Met Lys Thr Phe Cys Gly Thr Pro Glu Tyr Leu Ala Pro Glu Val
   305 310 315 320
   Leu Glu Asp Asn Asp Tyr Gly Arg Ala Val Asp Trp Trp Gly Leu Gly
   325 330 335
   Val Val Met Tyr Glu Met Met Cys Gly Arg Leu Pro Phe Tyr Asn Gln
   340 345 350
   Asp His Glu Lys Leu Phe Glu Leu Ile Leu Met Glu Glu Ile Arg Phe
   355 360 365
   Pro Arg Thr Leu Gly Pro Glu Ala Lys Ser Leu Leu Ser Gly Leu Leu
   370 375 380
   Lys Lys Asp Pro Lys Gln Arg Leu Gly Gly Gly Ser Glu Asp Ala Lys
   385                 390                 395                 400
   Glu Ile Met Gln His Arg Phe Phe Ala Gly Ile Val Trp Gln His Val
                   405                 410                 415
   Tyr Glu Lys Lys Leu Ser Pro Pro Phe Lys Pro Gln Val Thr Ser Glu
               420                 425                 430
   Thr Asp Thr Arg Tyr Phe Asp Glu Glu Phe Thr Ala Gln Met Ile Thr
           435                 440                 445
   Ile Thr Pro Pro Asp Gln Asp Asp Ser Met Glu Cys Val Asp Ser Glu
       450                 455                 460
   Arg Arg Pro His Phe Pro Gln Phe Ser Tyr Ser Ala Ser Ser Thr Ala
   465                 470                 475                 480
   *
(2) INFORMATION FOR SEQ ID NO: 3:
   (i) SEQUENCE CHARACTERISTICS:
      (A) LENGTH: 9 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO: 3:
   CTTGATGAG 9
(2) INFORMATION FOR SEQ ID NO: 4:
   (i) SEQUENCE CHARACTERISTICS:
      (A) LENGTH: 16 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO: 4:
   AATTCTCATC AAGCCC 16
(2) INFORMATION FOR SEQ ID NO: 5:
   (i) SEQUENCE CHARACTERISTICS:
      (A) LENGTH: 13 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO: 5:
   GATCCGCTGG AGC 13
(2) INFORMATION FOR SEQ ID NO: 6:
   (i) SEQUENCE CHARACTERISTICS:
      (A) LENGTH: 11 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO: 6:
   TAGCTCCAGC G   11
(2) INFORMATION FOR SEQ ID NO: 7:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO: 7:
   GTGGCTGACG GCCTCTGAG 19
(2) INFORMATION FOR SEQ ID NO: 8:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO: 8:
   AATTCTCAGA GGCCGTCAGC CACAGT 26
(2) INFORMATION FOR SEQ ID NO: 9:
   (i) SEQUENCE CHARACTERISTICS:
      (A) LENGTH: 9 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO: 9:
   CTTGATGAG 9
(2) INFORMATION FOR SEQ ID NO: 10:
   (i) SEQUENCE CHARACTERISTICS:
      (A) LENGTH: 16 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO: 10:
   AATTCTCATC AAGCCC 16
(2) INFORMATION FOR SEQ ID NO: 11:
   (i) SEQUENCE CHARACTERISTICS:
      (A) LENGTH: 34 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO: 11:
   GGCACCGGAT CCGACGTGGC TATTGTGAAG GAGG 34
(2) INFORMATION FOR SEQ ID NO: 12:
   (i) SEQUENCE CHARACTERISTICS:
      (A) LENGTH: 34 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO: 12:
   CTCCTGAATT CAGAGGCCGT CAGCCACAGT CTGG 34
(2) INFORMATION FOR SEQ ID NO: 13:
   (i) SEQUENCE CHARACTERISTICS:
      (A) LENGTH: 13 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO: 13:
   GATCCGCTGG AGC 13
(2) INFORMATION FOR SEQ ID NO: 14:
   (i) SEQUENCE CHARACTERISTICS:
      (A) LENGTH: 11 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO: 14:
   TAGCTCCAGC G 11
(2) INFORMATION FOR SEQ ID NO: 15:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO: 15:
   AATTCTCACT TCTTGAGGCC GTCAGCCACA GT 32
(2) INFORMATION FOR SEQ ID NO: 16:
   (i) SEQUENCE CHARACTERISTICS:
      (A) LENGTH: 13 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO: 16:
   CTGCAAAAGA CGG 13
(2) INFORMATION FOR SEQ ID NO: 17:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO: 17:
   CGCTCCGTCT TTTGCAG 17
(2) INFORMATION FOR SEQ ID NO: 18:
   (i) SEQUENCE CHARACTERISTICS:
      (A) LENGTH: 37 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO: 18:
   GTGGCTGACG GCCTCAAGAA GCAGAAGAAG AAGTGAG 37
(2) INFORMATION FOR SEQ ID NO: 19:
   (i) SEQUENCE CHARACTERISTICS:
      (A) LENGTH: 44 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO: 19:
   AATTCTCACT TCTTCTTCTG CTTCTTGAGG CCGTCAGCCA CAGT 44
(2) INFORMATION FOR SEQ ID NO: 20:
   (i) SEQUENCE CHARACTERISTICS:
      (A) LENGTH: 12 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (iv) ANTI-SENSE: NO
   (xi) SEQUENCE DESCRIPTION: SEQ ID NO: 20:
   Trp Thr Thr Ala Ile Gln Thr Val Ala Asp Gly Leu
   1               5                   10
(2) INFORMATION FOR SEQ ID NO: 21:
   (i) SEQUENCE CHARACTERISTICS:
      (A) LENGTH: 14 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (iv) ANTI-SENSE: NO
   (xi) SEQUENCE DESCRIPTION: SEQ ID NO: 21:
   Trp Thr Thr Ala Ile Gln Thr Val Ala Asp Gly Leu Lys Lys
   1               5                   10
(2) INFORMATION FOR SEQ ID NO: 22:
   (i) SEQUENCE CHARACTERISTICS:
      (A) LENGTH: 32 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (iv) ANTI-SENSE: NO
   (xi) SEQUENCE DESCRIPTION: SEQ ID NO: 22:
   Trp Thr Thr Ala Ile Gln Thr Val Ala Asp Gly Leu Lys Lys Gln Glu
   1               5                   10                  15
   Glu Glu Glu Met Asp Phe Arg Ser Gly Ser Pro Asp Asn Ser Gly Ala
               20                  25                  30
(2) INFORMATION FOR SEQ ID NO: 23:
   (i) SEQUENCE CHARACTERISTICS:
      (A) LENGTH: 18 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (iv) ANTI-SENSE: NO
   (xi) SEQUENCE DESCRIPTION: SEQ ID NO: 23:
   Trp Thr Thr Ala Ile Gln Thr Val Ala Asp Gly Leu Lys Lys Gln Lys
   1               5                   10                  15
   Lys Lys